# EUROPEAN PATENT SPECIFICATION

(11) **EP 1 451 287 B1**
(45) Date of publication and mention of the grant of the patent: **21.07.2010**
(21) Application number: 02806751.0
(22) Date of filing: 22.11.2002
(51) Int. Cl.: C12M 1/00

(54) **DEVICE FOR PRODUCTION OF ALGAE**
VORRICHTUNG ZUR ALGENPRODUKTION
DISPOSITIF POUR LA PRODUCTION D'ALGUES

(30) Priority: 22.11.2001 NO 20015705
(43) Date of publication of application: 01.09.2004
(73) Proprietor: Vaktek AS, 3292 Stavern (NO)
(72) Inventor: HEGG, Torbj rn, N-3292 Stavern (NO); SNEKKENES, Torbj rn, A., N-3290 Stavern (NO); SLETSJ E, Aage, N-3264 Larvik (NO)
(74) Representative: Bucks, Teresa Anne
(86) International application number: PCT/NO2002/000436
(87) International publication number: WO 2003/066799

(56) References cited:
- FR-A1- 2 596 412
- US-A- 4 253 271
- US-A- 5 981 271
- DATABASE WPI Week 199334, Derwent Publications Ltd., London, GB; AN 1993-269022, XP002977321 & JP 5 184 347 A (DAINIPPON INK. & CHEM. KK) 27 July 1993

## Description

The invention relates to a device for production of algae in a greenhouse.

The production of biomass such as algae has received attention in recent times in connection with its potential for utilisation as an article of food, medication and for energy production. When photosynthesis takes place in algae, light energy is converted by means of CO₂ and water to stored chemical energy by the production of cell material. Algal growth requires a medium consisting of water, mineral nutrients and CO₂. Continuous production of biomass of defined quality is the basis for industrial utilisation of microalgae. The practical use of algae includes several different groups of algae, such as, e.g., photosynthetic prokaryote and eukaryote micro algae.

Algae have traditionally been cultivated in open ponds containing a cultivation medium. The algae in the upper part of the culture fluid are supplied with sunlight and CO₂, thus enabling the photosynthesis to be implemented and the growth stimulated. To enable the algae in the lower parts of the fluid to also receive a sufficient supply, the fluid is stirred, e.g. by means of motor-driven water wheels.

Since algal growth is highly dependent on sunlight, there have been problems associated with the varying length of the days in Norway and in other northern countries. Good conditions are to be expected during the summer half of the year when the days are long, but substantially less yield in the winter half of the year, when outdoor cultivation will also have to be stopped on account of the low temperatures.

A similar problem is associated with the considerable temperature variations, since optimal algal growth is only achieved within a small temperature range.

WO 96/23865 discloses a production process for algae comprising a hydraulic closed loop in which a fluid containing the algae can circulate. The temperature of the algal fluid is kept within two predetermined values and the fluid is supplied with carbon dioxide and nutrients. A part of the loop is transparent to sunlight in order to stimulate photosynthesis.

This is a relatively complicated system and will not be satisfactory during periods with little sunlight. In a substantial part of the loop the algae are not exposed to light, resulting in reduced production.

A solution may be to make an indoor production plant with artificial lighting and climate control.

This will be extremely expensive in countries such as Norway where large amounts of energy will have to be supplied during a major part of the year. It is therefore desirable to find a method of controlling the climate in the production plants that have a rational energy consumption. Plants of this kind with a controlled climate are known from plant cultivation in greenhouses. See, e.g. the applicant's patent NO 146116, Device for a greenhouse or growth area for promoting the growth of plants or germination of seeds. The controlled supply of conditioned air is used here to provide a zone with air in optimal condition near the plants' growth areas.

This system, however, is not suitable for cultivating algae since algae require a completely different growth medium to that for plants. Algae are cultivated in a culture fluid, which requires stirring in order to provide identical conditions for all the algae in the culture. Nor is it obvious how the supply of conditioned air should be implemented, since containers for fluid with holes cannot be equipped in the same way as that described for a plant greenhouse.

The object of the invention is therefore to provide a device for production of algae in a greenhouse-like environment that provides good algal growth with little energy consumption.

This object is achieved by means of the features in the patent claims.

The device according to the invention comprises a house, preferably a greenhouse of the type used for plants (NO146116). The house is airtight. Inside the house is a reservoir for a cultivation medium/culture fluid. Above the reservoir are located a number of channels, which are preferably sloping. In a preferred embodiment the angle of inclination of the channels may be altered by means of a tilting device. In connection with the reservoir and the channels there is also at least one pump of a known type, e.g. a membrane pump. In an embodiment the reservoir may be composed of pipelines that connect the upper and lower ends of the channels and are connected to the pump. In connection with the house there are also devices for injection of air and for admixture of additives such as, e.g. CO₂ into the injected air.

Parts of the culture fluid are pumped up into the end of the highest-located channels, with the result that the culture fluid flows along the channels and back into the reservoir. By controlling the amount of culture fluid that is pumped up into the channels, the thickness of the fluid layer can be controlled. With a thin fluid layer (in the range <5mm) and a suitable angle of inclination, a wave train of small waves is produced, creating a rotating motion of the water molecules in the water layer (see Rouse, H, "Engineering Hydraulics", John Wiley & Sons, 1950, pp. 711-768). This rotation increases the number of algae units in the fluid that comes into contact with the light and the air and exchanges CO₂ and O₂.

The channels are located close together, thus forming separate spaces above and below the channels. Between the channels are provided openings of a defined size. In connection with the space below the channels, there is a device for injecting air into this space. The device may, e.g., be a fan where the fan propeller may have adjustable blades, or the speed can be regulated to enable the air pressure out from the fan into the space below the channels to be adjusted. When air is injected into the space below the channels, an overpressure is created that forces the air up through the openings between the channels into the space above the channels. By controlling the addition of CO₂ and any other additives as well as the temperature of the air forced up between the channels, highly favourable conditions for algal growth can be obtained. By means of precise control of the air pressure in the lower space and thereby of the amount and pressure of the air rising up between the channels, a zone is formed above the channels with these favourable conditions. Energy savings are thereby achieved compared with having these conditions in the entire air space above the channels. Since the warm, moist air does not rise up to the ceiling of the greenhouse, condensation is also avoided, thereby preventing dimming of the incoming sunlight and permitting it to be fully exploited. In summer the rising air will create a cooled air cushion that protects the algal culture against overheating, and unnecessary energy is not used for cooling the whole space above the channels.

The invention will now be described in greater detail by means of an embodiment with reference to the accompanying drawings.
Figure 1 is a view of a greenhouse according to the invention, equipped for cultivation of algae.
Figure 2 illustrates an embodiment of channels and a reservoir for culture fluid viewed from a longitudinal side.
Figure 3 illustrates the same embodiment as in figure 2, but viewed from a short end.

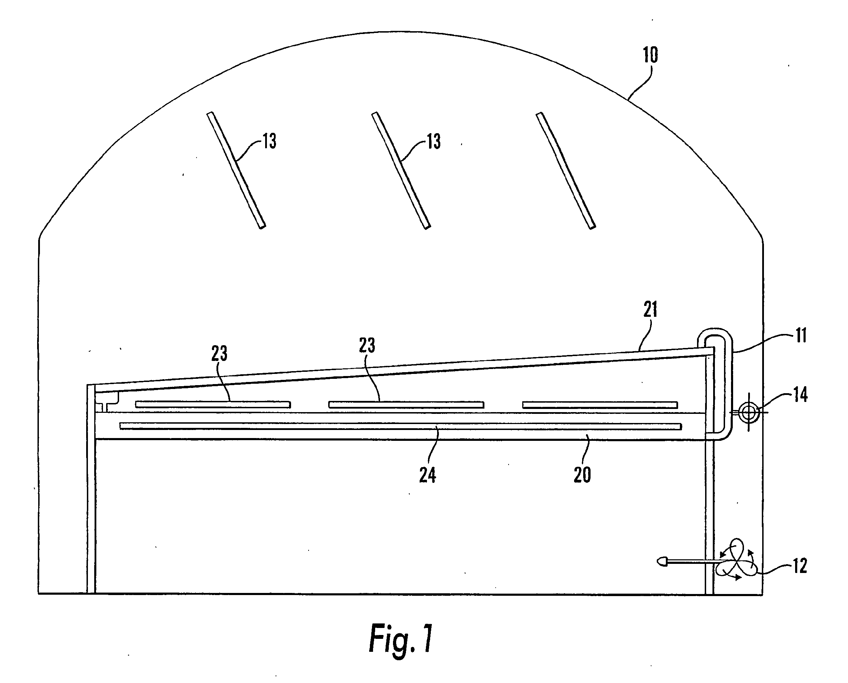

The greenhouse illustrated in figure 1 is of a similar type to that used for cultivation of plants. The ceiling is transparent to visible light, preferably to those wavelengths that best stimulate the photosynthesis of the algae. This will depend on the type of algae that are to be cultivated, and may, e.g., be in the range 600-700nm, or more specifically 675nm ± 5% for blue-green algae that absorb light in the red area of the light's wavelength spectrum. Channels 21 are located slantingly inside the house. A reservoir 20 for a culture fluid is located below the channels, and is preferably in the form of open gutters, but the reservoir may also be composed of a closed pipe system that is filled with culture fluid. In both cases a pump 14 is provided that pumps the culture fluid up via pipe 11 to the highest end of the channels for circulation of the culture fluid. The culture fluid then flows down along the channels back into the reservoir.

Algal growth requires a medium, the culture fluid, consisting of water, mineral nutrients and CO₂. Waste water contains a number of mineral nutrients and in a preferred embodiment waste water is employed as culture fluid for the algae. The algae consume minerals and also convert some of them to other substances, which has the effect of purifying the waste water. The use of algal cultivation may thereby be envisaged as part of a purification process for waste water where the waste water is removed from the ordinary purification plants, e.g. after physical filtration, but before the addition of any purifying chemicals. When the algae have consumed the necessary nutrients, the waste water can be returned to the purification plant for further treatment. This could therefore result in a reduction in the amount of purifying chemicals in such purification plants.

The object of the inclined arrangement of the channels is to create waves in the fluid flowing along the channels. Such waves are produced at a certain velocity and layer thickness of the fluid layer flowing in the channels (see Rouse, H, "Engineering Hydraulics", John Wiley & Sons, 1950, pp. 711-768). The angle of inclination of the channels can therefore be varied, depending on the thickness of the layer of culture fluid flowing in the channels.

The channels are located close together, forming a partition in the house, i.e. causing the formation of separate spaces above and below the channels.

In areas with varying length of daylight, it has to be supplemented with artificial light during parts of the 24-hour period. The artificial light is adapted to the previously mentioned wavelength of, e.g., 675nm ± 5% for the best possible stimulation of algal growth. The light sources may be placed in alternative positions, the various alternatives being illustrated in figure 1. 13 indicates a lighting fixture arranged above the channels in a manner known from greenhouses for plants. The fixture may be positioned so as to avoid as far as possible blocking the natural light when it is at its strongest. 23 indicates a lighting fixture arranged below the channels. In this case the channels are produced in a transparent material, with the result that the culture fluid is illuminated directly from below. In the preferred embodiment with a culture fluid reservoir in open gutters, in this case the lighting fixture is placed between the channels and the gutters and the fluid in the gutters is thereby also illuminated. This is the embodiment that best exploits natural and artificial light simultaneously and is most energy-efficient since practically all light and heat are transferred to the culture fluid. As a third embodiment the light source may also be submerged in the culture fluid reservoir, as illustrated in at 24 in figure 1.

A fan device 12 blows air into the space below the channels. For optimal growth, it is desirable to optimise the CO₂ content in the air in which the algae are growing. For plants the CO₂ concentration is 500-1000 ppm, optimally 800 ppm, and it is assumed that similar conditions apply to algae. The injected air is regulated to the desired temperature, preferably in the range 20-30°C and CO₂ is added in the correct mixture ratio, 500-1000 ppm, preferably 800 ppm, together with any other additives such as, e.g. phosphate, nitrate and ammonium.

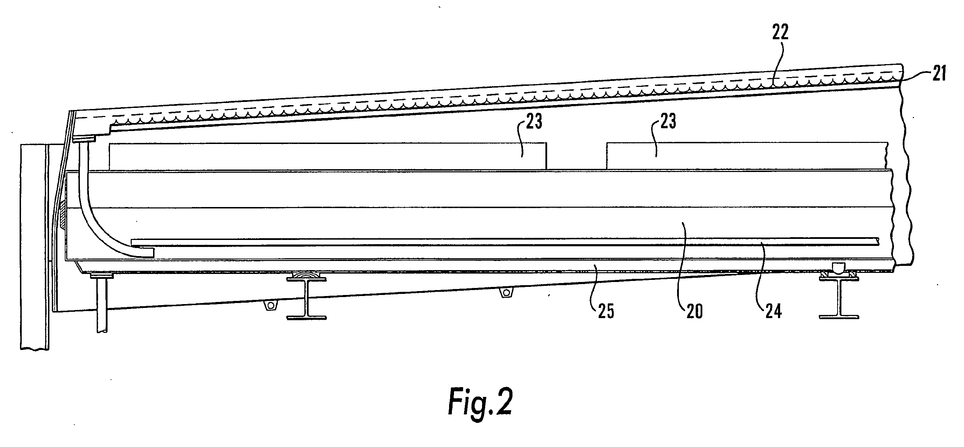

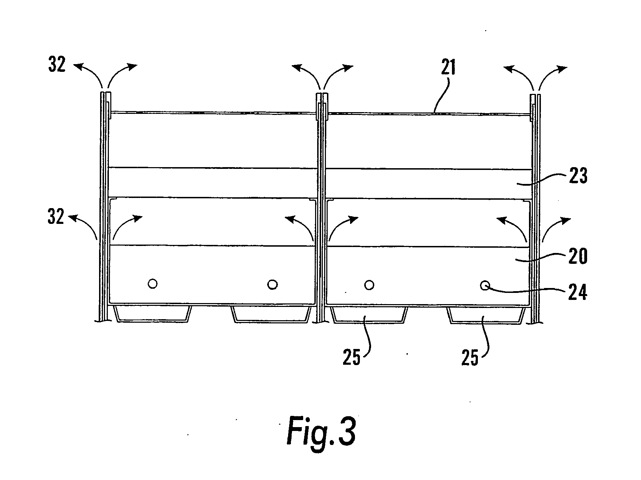

Figures 2 and 3 illustrate sections through the channels viewed from different sides. 22 indicates waves on the surfaces of the channels when the culture fluid runs down along the channels. Those parts in the figures that are the same as in figure 1 have the same reference numerals. These figures illustrate yet another preferred embodiment of the invention, where additional gutters 25 are provided below the culture fluid reservoir channels. These additional gutters 25 convey temperate water for cooling or heating of the culture fluid to the optimal growth temperature for the algae, which is approximately 20-35°C.

When air is injected into the space below the channels, an overpressure is created there. Between the channels is provided a duct system/openings 32 (fig. 3). The overpressure below the channels causes the air to be forced up through the duct system/openings 32 between the channels, illustrated by arrows in figure 3. By regulating the velocity of the injected air in the space below the channels, e.g. by changing the angles of the fan blades, the overpressure in the space is also regulated and thereby the amount of air that is forced up between the channels. The design of the gutter system/openings 32 and the extent of the overpressure in the space below the channels interact so as to cause the flow of air forced up between the channels to prevent cold air from sinking down to the fluid surface, but is sufficiently weak to only cause a slow stirring in the layer of air directly above the fluid surface. This results in the formation of a defined layer of conditioned air above the culture fluid in the channels. In this fashion very good control is obtained of the cultivation conditions, while it is only necessary to have optimal conditions in this defined layer, and not in the whole house, thus providing a substantial energy saving effect. When the outside temperatures are low, warm, moist air is prevented from rising and condensing, and perhaps even freezing, on the inside of the ceiling and thereby preventing natural light from penetrating. This also provides an energy saving effect.

## Claims

1. A device for production of algae comprising a greenhouse (10), a reservoir (20) for culture fluid and a pump device (14) for circulation of culture fluid, **characterised in that**
- channels (21) for transport of culture fluid are provided in the greenhouse, parallel to one another and at such a height that the house is divided by the channels (21) into an upper and a lower space,
- a supply device for culture fluid is provided at one end of the channels and an outlet at the other end and the flow of culture fluid is impelled by means of the pump device (14),
- the device also comprises a fan arrangement (12) for blowing air into the bottom space and that between the channels are openings (32) of a defined size for the passage of air, and
- lighting (13, 23, 24) is provided for illumination of the channels.

2. A device according to claim 1,
**characterised in that** the channels (21) are sloping between input and output at an angle that is variable.

3. A device according to claim 2,
**characterised in that** the angle varies with the amount of culture fluid flowing thereon in order to obtain waves in the culture fluid in the channels (21).

4. A device according to claim 1,
**characterised in that** the culture fluid reservoir (20) and the channels (21) are located in the same area in the house.

5. A device according to claim 1,
**characterised in that** the culture fluid reservoir comprises a closed pipe system.

6. A device according to claim 1,
**characterised in that** it also comprises artificial lighting, which is preferably located above or below the channels.

7. A device according to claim 6,
**characterised in that** the artificial lighting (24) is located in the culture fluid reservoir (20).

8. A device according to one of the claims 6 or 7,
**characterised in that** the artificial lighting predominantly comprises light with a wavelength of 675nm± 5%.

9. A device according to one of the claims 1-8,
**characterised in that** the ceiling of the house (10) is transparent to visible light.

10. A device according to one of the claims 1-8,
**characterised in that** the ceiling of the house (10) has good transmission properties for light with a wavelength of 675nm± 5%.

11. A device according to one of the claims 1-12,
**characterised in that** it also comprises a set of gutters (25) in or below the culture fluid reservoir (20), containing a fluid that has good thermal contact with the culture fluid reservoir.

12. A device according to claim 11,
**characterised in that** the temperature of the fluid in the gutters (25) can be regulated in order to control the temperature in the culture fluid reservoir (20).

13. A device according to claim 1,
**characterised in that** the incoming air is mixed with CO₂ before injection, preferably with a concentration of CO₂ at 800 ppm.

## Patentansprüche

1. Einrichtung zur Algenproduktion umfassend ein Treibhaus (10), einen Behälter (20) für eine Nährlösung und eine Pumpeinrichtung (14) für die Zirkulation der Nährlösung, **dadurch gekennzeichnet, dass**
- Kanäle (21) für den Transport der Nährlösung in dem Treibhaus parallel zueinander und in einer solchen Höhe vorgesehen sind, dass das Haus durch die Kanäle (21) in einen oberen und einen unteren Raum geteilt ist,
- eine Versorgungseinrichtung für die Nährlösung an einem Ende der Kanäle und ein Auslass an dem anderen Ende vorgesehen ist und der Fluss der Nährlösung mittels der Pumpeinrichtung (14) angetrieben ist,
- wobei die Einrichtung ebenfalls eine Gebläseanordnung (12) für das Einblasen von Luft in den Bodenraum umfasst und dass zwischen den Kanälen Öffnungen (32) einer definierten Größe für den Luftdurchlass vorhanden sind, und
- eine Beleuchtung (13, 23, 24) für die Ausleuchtung der Kanäle vorgesehen ist.

2. Einrichtung nach Anspruch 1, **dadurch gekennzeichnet, dass** die Kanäle (21) zwischen Einlass und Auslass in einem Winkel geneigt sind, der variabel ist.

3. Einrichtung nach Anspruch 2, **dadurch gekennzeichnet, dass** der Winkel mit der Menge der Nährlösung variiert, die darauf fließt, so dass in der Nährlösung in den Kanälen (21) Wellen erhalten werden.

4. Einrichtung nach Anspruch 1, **dadurch gekennzeichnet, dass** der Nährlösungsbehälter (20) und die Kanäle (21) in demselben Bereich in dem Haus angeordnet sind.

5. Einrichtung nach Anspruch 1, **dadurch gekennzeichnet, dass** der Nährlösungsbehälter ein geschlossenes Rohrsystem umfasst.

6. Einrichtung nach Anspruch 1, **dadurch gekennzeichnet, dass** sie ebenfalls eine künstliche Beleuchtung umfasst, welche vorzugsweise über oder unter den Kanälen angeordnet ist.

7. Einrichtung nach Anspruch 6, **dadurch gekennzeichnet, dass** die künstliche Beleuchtung (24) in dem Nährlösungsbehälter (20) angeordnet ist.

8. Einrichtung nach einem der Ansprüche 6 oder 7, **dadurch gekennzeichnet, dass** die künstliche Beleuchtung vorwiegend ein Licht mit einer Wellenlänge von 675 nm± 5% umfasst.

9. Einrichtung nach einem der Ansprüche 1-8, **dadurch gekennzeichnet, dass** das Dach des Hauses (10) für sichtbares Licht transparent ist.

10. Einrichtung nach einem der Ansprüche 1-8, **dadurch gekennzeichnet, dass** das Dach des Hauses (10) gute Übertragungseigenschaften für Licht mit einer Wellenlänge von 675 nm± 5% aufweist.

11. Einrichtung nach einem der Ansprüche 1-12, **dadurch gekennzeichnet, dass** sie ebenfalls eine Gruppe Rinnen (25) in oder unter dem Nährlösungsbehälter (20) umfasst, die ein Fluid umfassen, die einen guten thermischen Kontakt mit dem Nährlösungsbehälter aufweist.

12. Einrichtung nach Anspruch 11, **dadurch gekennzeichnet, dass** die Temperatur des Fluids in den Rinnen (25) reguliert werden kann, so dass die Temperatur in dem Nährlösungsbehälter (20) gesteuert wird.

13. Einrichtung nach Anspruch 1, **dadurch gekennzeichnet, dass** die eingehende Luft vor dem Einblasen mit CO₂ gemischt wird, vorzugsweise mit einer CO₂-Konzentration von 800 ppm.

## Revendications

1. Dispositif de production d'algues comprenant une serre (10), un réservoir (20) pour un fluide de culture et un dispositif formant pompe (14) pour la circulation du fluide de culture, **caractérisé en ce que**
- des canaux (21) pour le transport du fluide de culture sont prévus dans la serre, parallèles les uns aux autres et à une hauteur telle que la serre est divisée par les canaux (21) en un espace supérieur et un espace inférieur,
- un dispositif d'alimentation pour le fluide de culture est prévu à une extrémité des canaux et une sortie est prévue à l'autre extrémité, et la circulation du fluide de culture est forcée au moyen du dispositif formant pompe (14),
- le dispositif comprend également un agencement de ventilateur (12) pour souffler de l'air dans l'espace inférieur, et **en ce que**, entre les canaux, il y a des ouvertures (32) d'une taille définie pour le passage d'air, et
- un éclairage (13, 23, 24) est prévu pour éclairer les canaux.

2. Dispositif selon la revendication 1, **caractérisé en ce que** les canaux (21) sont inclinés entre l'entrée et la sortie d'un angle variable.

3. Dispositif selon la revendication 2, **caractérisé en ce que** l'angle varie avec la quantité de fluide de culture circulant sur celui-ci afin d'obtenir des ondes dans le fluide de culture dans les canaux (21).

4. Dispositif selon la revendication 1, **caractérisé en ce que** le réservoir de fluide de culture (20) et les canaux (21) sont situés dans la même zone dans la serre.

5. Dispositif selon la revendication 1, **caractérisé en ce que** le réservoir de fluide de culture comprend un système de tuyaux fermé.

6. Dispositif selon la revendication 1, **caractérisé en ce qu'**il comprend également un éclairage artificiel, qui est de préférence situé au-dessus ou au-dessous des canaux.

7. Dispositif selon la revendication 6, **caractérisé en ce que** l'éclairage artificiel (24) est situé dans le réservoir de fluide de culture (20).

8. Dispositif selon l'une des revendications 6 ou 7, **caractérisé en ce que** l'éclairage artificiel comprend principalement une lumière avec une longueur d'onde de 675 nm ± 5 %.

9. Dispositif selon l'une des revendications 1 à 8, **caractérisé en ce que** le plafond de la serre (10) laisse passer la lumière visible.

10. Dispositif selon l'une des revendications 1 à 8, **caractérisé en ce que** le plafond de la serre (10) a de bonnes propriétés de transmission pour la lumière avec une longueur d'onde de 675 nm ± 5 %.

11. Dispositif selon l'une des revendications 1 à 12, **caractérisé en ce qu'**il comprend également un ensemble de gouttières (25) dans ou au-dessous du réservoir de fluide de culture (20), contenant un fluide qui a un bon contact thermique avec le réservoir de fluide de culture.

12. Dispositif selon la revendication 11, **caractérisé en ce que** la température du fluide dans les gouttières (25) peut être régulée afin de commander la température dans le réservoir de fluide de culture (20).

13. Dispositif selon la revendication 1, **caractérisé en ce que** l'air entrant est mélangé avec du CO₂ avant injection, de préférence avec une concentration de CO₂ à 800 ppm.
